# EUROPÄISCHE PATENTSCHRIFT

(11) **EP 0 423 595 B1**
(45) Veröffentlichungstag und Bekanntmachung des Hinweises auf die Patenterteilung: **18.05.1994**
(21) Anmeldenummer: 90119316.9
(22) Anmeldetag: 09.10.1990
(51) Int. Cl.: C07D 213/26

(54) **Verfahren zur Herstellung von 3-Trichlormethyl-pyridin**
Process for the preparation of 3-trichloromethyl pyridine
Procédé pour la préparation de 3-trichlorométhyl pyridine

(30) Priorität: 20.10.1989 DE 3934957
(43) Veröffentlichungstag der Anmeldung: 24.04.1991
(73) Patentinhaber: BAYER AG, 51368 Leverkusen (DE)
(72) Erfinder: Jelich, Klaus, Dr., W-5600 Wuppertal 1 (DE); Lindel, Hans, Dr., W-5090 Leverkusen 1 (DE)

(56) Entgegenhaltungen:
- EP-A- 0 065 358
- FR-A- 1 394 362
- US-A- 3 412 095
- CHEMICAL ABSTRACTS, Band 105, Nr. 21, 24. November 1986, Seite 709, Zusammenfassung Nr. 190869x, Columbus, Ohio, US; E.G. NOVIKOV et al.: "Beta-picoline fraction and sulfates of pyridine series bases"

## Beschreibung

Die Erfindung betrifft ein Verfahren zur Herstellung von 3-Trichlormethyl-pyridin, welches als Zwischenprodukt zur Herstellung von bekannten Herbiziden und Insektiziden verwendet werden kann.

Es ist bekannt, daß man 3-Trichlormethyl-pyridin erhält, wenn man Nicotinsäure mit Phosphor(V)-chlorid auf 115° C erhitzt (vgl. J. Chem. Soc. Perkin Trans I, 1989, 283-287) oder mit Phenyldichlorphosphan (C₆H₅PCl₂), Phosphor(III)-chlorid und Chlor bei Temperaturen zwischen 100° C und 250° C umsetzt (vgl. US-P 4634771). Die hierbei erreichbaren Ausbeuten sind jedoch nicht zufriedenstellend; außerdem ist die Verwendung von Phosphorverbindungen wegen der damit verbundenen Abwasserbelastung problematisch.

Weiter ist bekannt, das man 3-Trichlormethyl-pyridin auch durch Umsetzung von 3-Methyl-pyridin mit Chlor in Gegenwart eines Metalloxid- oder Metallchlorid-Katalysators bei Temperaturen zwischen 200° C und 350° C erhalten kann (vgl. EP-A 65358). Die Reaktion verläuft jedoch nicht einheitlich und die entstehenden Produktgemische sind nur schwierig aufzutrennen.

Ferner ist bekannt, daß 3-Methyl-pyridin auch durch Chlorierung in einem Schwefelsäure-Oleumgemisch bei 70° C bis 75° C unter Belichtung in 3-Trichlormethyl-pyridin überführt werden kann (vgl. FR-A-1394362). Es hat sich jedoch gezeigt, daß auch hierbei schwer aufzutrennende Produktgemische entstehen.

Es wurde nun gefunden, daß man 3-Trichlormethyl-pyridin der Formel (I) 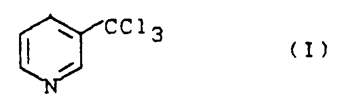
in sehr guter Ausbeute und in hoher Reinheit erhält, wenn man 3-Methyl-pyridin-Hydrogensulfat der Formel (II) 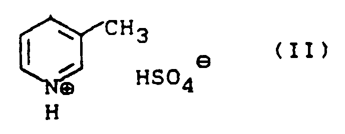
mit elementarem Chlor unter Belichtung mit sichtbarem und/oder ultraviolettem Licht, gegebenenfalls in Gegenwart eines Katalysators, bei Temperaturen zwischen 70° C und 150° C umsetzt.

Überraschenderweise kann 3-Trichlormethyl-pyridin nach dem erfindungsgemäßen Verfahren auf relativ einfache Weise in sehr guter Ausbeute und in hoher Reinheit hergestellt werden, während nach den bekannten Verfahren dieses Produkt in mäßigen Ausbeuten und in unbefriedigender Qualität erhalten wird.

Vorteile des erfindungsgemäßen Verfahrens liegen einerseits in der Verwendbarkeit des im Vergleich mit Nicotinsäure wesentlich preisgünstigeren 3-Methyl-pyridins als Basisverbindung, andererseits in der geringen Abwasserbelastung im Vergleich mit der Chlorierung in Schwefelsäure/Oleum verbunden mit einer hohen Raum-Zeit-Ausbeute.

Der Ablauf des erfindungsgemäßen Verfahrens kann durch das folgende Formelschema skizziert werden: 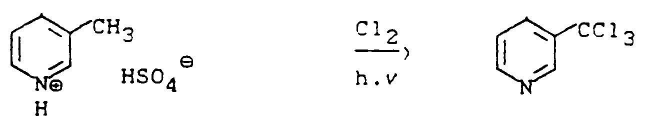
3-Methylpyridin-Hydrogensulfat der Formel (II) wird durch Zusammengeben von (angenähert) äquimolaren Mengen 3-Methyl-pyridin und Schwefelsäure, gegebenenfalls unter Verwendung eines Verdünnungsmittels, wie z.B. Benzol, Toluol, Essigsäureethylester und/oder Dioxan, bei Temperaturen zwischen 20° C und 100° C erhalten.

Das 3-Methyl-pyridin-Hydrogensulfat der Formel (II) kann in einer vorgeschalteten Reaktionsstufe erzeugt und als reines Produkt isoliert werden; es kann jedoch auch ohne Zwischenisolierung gemäß dem erfindungsgemäßen Verfahren mit Chlor umgesetzt werden.

Das erfindungsgemäße Verfahren wird gegebenenfalls in Gegenwart eines Katalysators durchgeführt. Es können praktisch alle für derartige Reaktionen geeigneten Katalysatoren verwendet werden. Für das erfindungsgemäße Verfahren vorzugsweise eingesetzte Katalysatoren sind Carbonsäureamide, insbesondere aromatische Carbonsäureamide wie z.B. Benzamid.

Das erfindungsgemäße Verfahren wird unter Belichtung durchgeführt. Es kommen hierbei praktisch alle für photochemische Reaktionen üblichen Belichtungsmittel in Betracht. Bevorzugte Lichtquellen sind beim erfindungsgemäßen Verfahren insbesondere solche, die Licht im Grenzbereich zwischen sichtbarem und ultraviolettem Licht emittieren. Es sind also auch normale Glühlampen zur Belichtung beim erfindungsgemäßen Verfahren geeignet.

Das erfindungsgemäße Verfahren wird im allgemeinen bei Temperaturen zwischen 90° C und 130° C durchgeführt. Das erfindungsgemäße Verfahren wird im allgemeinen unter Normaldruck durchgeführt. Es ist jedoch auch möglich, unter erhöhtem oder vermindertem Druck, vorzugsweise zwischen 0,1 und 10 bar zu arbeiten.

Zur Durchführung des erfindungsgemäßen Verfahrens setzt man auf 1 Mol 3-Methyl-pyridin-hydrogensulfat der Formel (II) im allgemeinen 3 bis 15 Mol, vorzugsweise 3 bis 10 Mol, Chlor ein.

3-Methyl-pyridin-hydrogensulfat der Formel (II) erzeugt, indem man 3-Methyl-pyridin vorlegt und etwa die äquimolare Menge Schwefelsäure bei Temperaturen zwischen 20° C und 100° C dazu gibt. Man stellt dann - gegebenenfalls nach Zugabe eines Katalysators - die erforderliche Reaktionstemperatur ein und leitet dann unter Belichtung langsam Chlor ein bis die Umsetzung praktisch abgeschlossen ist.

Nach Abkühlen wird mit Wasser und einem mit Wasser praktisch nicht mischbaren organischen Lösungsmittel, wie z.B. Essigsäureethylester, verdünnt und ein schwach alkalischer pH-Wert eingestellt. Die organische Phase wird abgetrennt und die wäßrige Phase gegebenenfalls nachextrahiert. Aus der organischen Phase erhält man nach Trocknen, z.B. mit Magnesiumsulfat, Filtrieren und Einengen des Filtrats einen Rückstand, welcher im wesentlichen das Produkt der Formel (I) enthält.

Das nach dem erfindungsgemäßen Verfahren herzustellende 3-Trichlormethyl-pyridin kann als Zwischenprodukt zur Herstellung von Herbiziden (vgl. EP-A 65358) oder von Insektiziden verwendet werden.

Beispielhaft sei eine typische Reaktionssequenz zur Herstellung eines insektiziden Wirkstoffs aufgeführt: 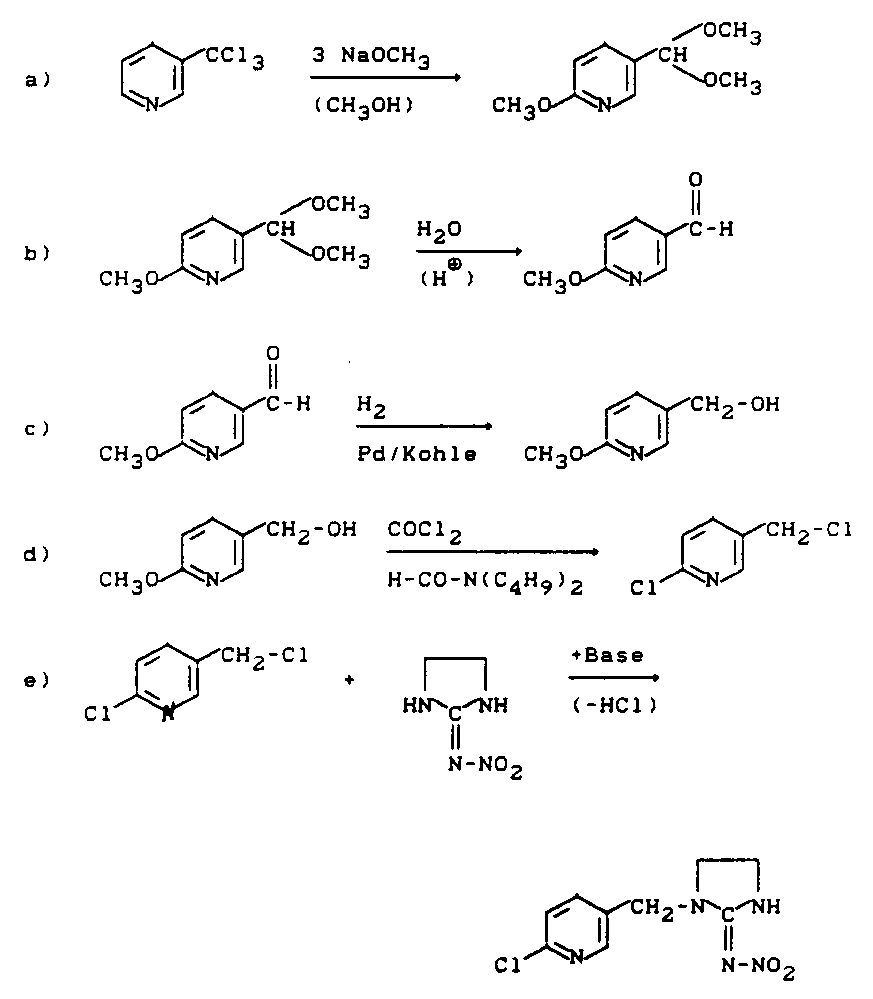
Reaktionsstufen a) bis einschließlich d) erfolgen hier gemäß der im Hinblick auf vorhergehende Anmeldung älteren, aber nicht vorveröffentlichten Deutschen Patentanmeldung P 38 42 359.

Reaktionsstufe e) erfolgt entsprechend der veröffentlichten Europäischen Patentanmeldung EP 0 192 060 A1.

Eine weitere Herstellungsmethode eines insektiziden Wirkstoffs sei im folgenden aufgeführt: 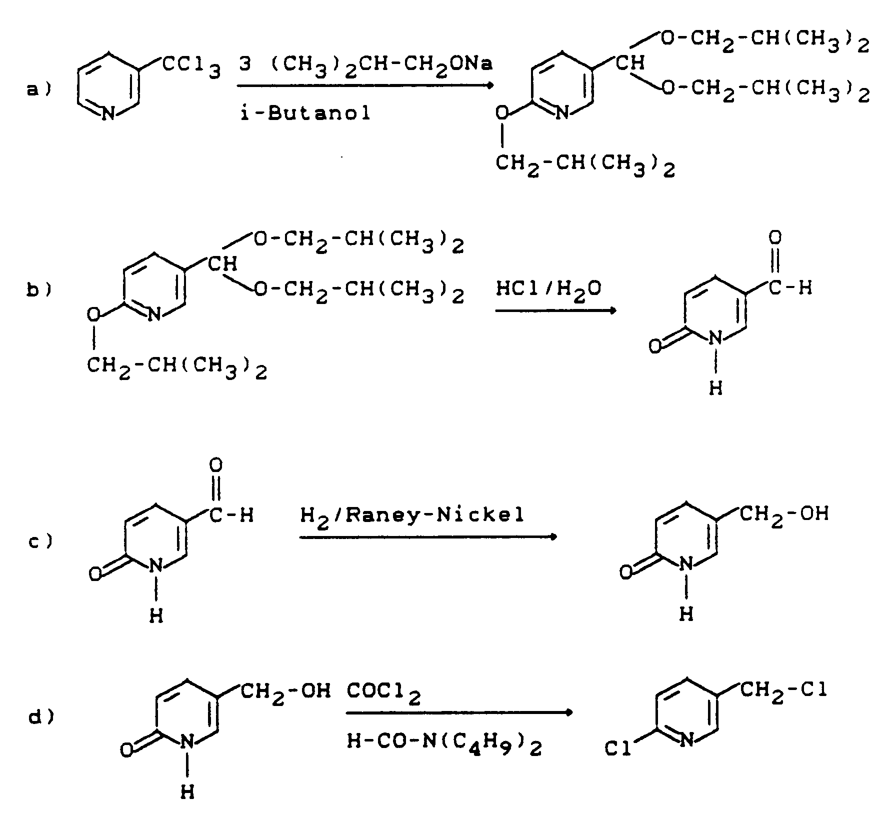 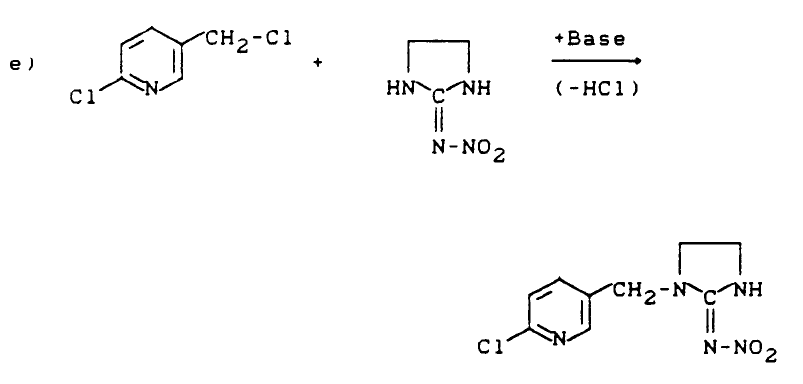
Reaktionsstufen a) bis einschließlich d) erfolgen hier gemäß der im Hinblick auf vorliegende Anmeldung älteren, aber nicht vorveröffentlichten Deutschen Patentanmeldung P 38 42 358.

Reaktionsstufe e) erfolgt entsprechend der veröffentlichten Europäischen Patentanmeldung EP 0 192 060 A1.

### Herstellungsbeispiel:

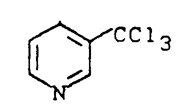

6,86 g (0,067 Mol) konz. (96%ige) Schwefelsäure wird zu 6,0 g (0,0645 Mol) 3-Methyl-pyridin tropfenweise so gegeben, daß durch die exotherme Reaktion eine Temperatur von 80° C erreicht und gehalten wird.

Anschließend werden 0,16 g (0,0013 Mol) Benzamid dazu gegeben und mit Hilfe einer Osram-300Watt-Glühlampe, die gleichzeitig als Licht- und Wärmequelle dient, das Gemisch auf 115° C bis 120° C erhitzt. Bei dieser Temperatur wird 14 Stunden lang ein schwacher Chlorstrom durch das als Schmelze vorliegende Reaktionsgemisch geleitet. Dann wird die Lichtquelle abgestellt, bei 70° C das Reaktionsgemisch mit Essigsäureethylester und anschließend mit Eiswasser verdünnt und mit Natriumcarbonat schwach alkalisch gestellt. Die organische Phase wird abgetrennt und die wäßrige Phase zweimal mit Essigester nachextrahiert. Die vereinigten organischen Phasen werden mit Magnesiumsulfat getrocknet und filtriert. Vom Filtrat wird das Lösungsmittel im Wasserstrahlvakuum sorgfältig abdestilliert.

Man erhält 10,7 g (83% der Theorie) 3-Trichlormethyl-pyridin als hellgelben, flüssigen Rückstand mit einem (gaschromatographisch bestimmten) Gehalt von 98%.

Das Produkt kann durch Vakuumdestillation weiter gereinigt werden (Siedepunkt: 105° C-107° C/15 mbar).

## Patentansprüche

1. Verfahren zur Herstellung von 3-Trichlormethyl-pyridin der Formel (I) 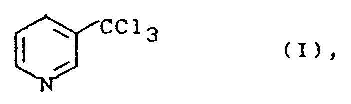 dadurch gekennzeichnet, daß man 3-Methyl-pyridin-Hydrogensulfat der Formel (II) 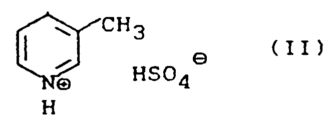 erzeugt, indem man 3-Methyl-pyridin vorlegt und etwa die äquimolare Menge Schwefelsäure bei Temperaturen zwischen 20° C und 100° C dazugibt, anschließend, gegebenenfalls nach Zugabe eines Katalysators, eine Reaktionstemperatur zwischen 90° C und 130° C einstellt und dann unter Belichtung mit sichtbarem und/oder ultraviolettem Licht langsam elementares Chlor einleitet und das erhaltene Reaktionsprodukt mit einem mit Wasser praktisch nicht mischbaren organischen Lösungsmittel extrahiert und nach an sich bekannten Methoden aufarbeitet.

2. Verfahren gemäß Anspruch 1, dadurch gekennzeichnet, daß in Gegenwart eines Carbonsäureamids als Katalysator gearbeitet wird.

3. Verfahren gemäß Anspruch 1 und 2, dadurch gekennzeichnet, daß in Gegenwart von Benzoesäureamid als Katalysator gearbeitet wird.

4. Verfahren gemäß Anspruch 1 bis 3, dadurch gekennzeichnet, daß als Lichtquellen solche eingesetzt werden, die im Grenzbereich zwischen sichtbarem und ultraviolettem Licht emittieren.

5. Verfahren gemäß Anspruch 1 bis 4, dadurch gekennzeichnet, daß die Umsetzung bei Temperaturen zwischen 90° C und 130° C durchgeführt wird.

6. Verfahren gemäß Anspruch 1 bis 6, dadurch gekennzeichnet, daß man zur Durchführung des Verfahrens pro 1 Mol 3-Methyl-pyridin-Hydrogensulfat der Formel (II) 3 bis 15 Mol elementares Chlor einsetzt.

## Claims

1. Process for the preparation of 3-trichloromethyl-pyridine, of the formula (I), 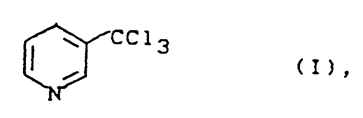 characterized in that 3-methyl-pyridine hydrogen sulphate, of the formula (II), 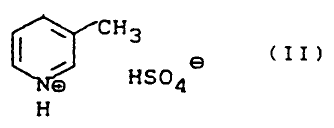 is prepared by introducing 3-methyl-pyridine into the reaction vessel and adding an approximately equimolar amount of sulphuric acid at temperatures between 20°C and 100°C, a reaction temperature between 90°C and 130°C is subsequently established, if appropriate after a catalyst has been added, elemental chlorine is then slowly passed in with exposure to visible and/or ultraviolet light, and the resulting reaction product is extracted with an organic solvent which is virtually immiscible with water, and the extract is worked up by methods known per se.

2. Process according to Claim 1, characterized in that it is carried out in the presence of a carboxamide as the catalyst.

3. Process according to Claims 1 and 2, characterized in that it is carried out in the presence of benzamide as the catalyst.

4. Process according to Claims 1 to 3, characterized in that the light sources employed are those which emit at the border between visible and ultraviolet light.

5. Process according to Claims 1 to 4, characterized in that the reaction is carried out at temperatures between 90°C and 130°C.

6. Process according to Claims 1 to 5, characterized in that, for carrying out the process, 3 to 15 moles of elemental chlorine are employed per mole of 3-methyl-pyridine hydrogen sulphate, of the formula (II).

## Revendications

1. Procédé de production de 3-trichlorométhylpyridine de formule (I) 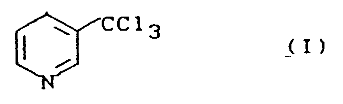 caractérisé en ce qu'on produit de l'hydrogénosulfate de 3-méthylpyridine de formule (II) 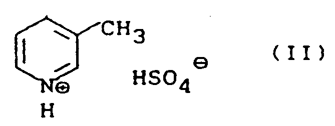 en partant de 3-méthylpyridine et en y ajoutant une quantité à peu près équimolaire d'acide sulfurique à des températures comprises entre 20°C et 100°C, en réglant ensuite, éventuellement après addition d'un catalyseur, une température de réaction comprise entre 90°C et 130°C puis en faisant passer lentement du chlore élémentaire sous éclairement avec de la lumière visible et/ou de la lumière ultraviolette et en extrayant le produit réactionnel obtenu avec un solvant organique pratiquement non miscible à l'eau et en le traitant ensuite par des procédés connus.

2. Procédé suivant la revendication 1, caractérisé en ce qu'on opère en présence d'un carboxamide comme catalyseur.

3. Procédé suivant les revendications 1 et 2, caractérisé en ce qu'on opère en présence de benzamide comme catalyseur.

4. Procédé suivant les revendications 1 à 3, caractérisé en ce qu'on utilise comme sources lumineuses des sources qui émettent de la lumière ultraviolette dans la région limitrophe entre la lumière visible et la lumière ultraviolette.

5. Procédé suivant les revendications 1 à 4, caractérisé en ce qu'on conduit la réaction à des températures comprises entre 90°C et 130°C.

6. Procédé suivant les revendications 1 à 5, caractérisé en ce qu'on utilise pour sa mise en oeuvre 3 à 15 moles de chlore élémentaire par mole d'hydrogénosulfate de 3-méthylpyridine de formule (II).
